(19) Europäisches Patentamt
European Patent Office
Office européen des brevets

(11) Publication number: **0 334 049 A2**

(12) **EUROPEAN PATENT APPLICATION**

(21) Application number: 89103249.2

(51) Int. Cl.4: C07C 63/68 , C07C 51/567

(22) Date of filing: 24.02.89

The title of the invention has been amended (Guidelines for Examination in the EPO, A-III, 7.3).

(30) Priority: 24.02.88 US 160033

(43) Date of publication of application: 27.09.89 Bulletin 89/39

(84) Designated Contracting States: AT BE CH DE ES FR GB IT LI NL SE

(71) Applicant: OCCIDENTAL CHEMICAL CORPORATION
P.O. Box 189
Niagara Falls New York 14302(US)

(72) Inventor: Spohn, Ronald F.
60 Charter Oaks
Williamsville New York(US)
Inventor: Sapienza, Frank J., Jr.
60 Partridge Run
West Amherst New York(US)
Inventor: Morth, Arthur H.
1181 Ransom Road
Grand Island New York(US)

(74) Representative: Vossius & Partner
Siebertstrasse 4 P.O. Box 86 07 67
D-8000 München 86(DE)

(54) **Process for the preparation of halogen-substituted phthalic anhydrides.**

(57) A process for the preparation of halogen substituted phthalic anhydrides of the formula:

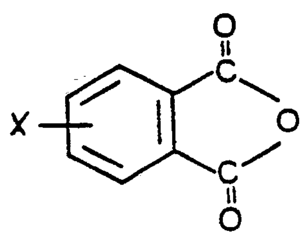

wherein X is a halogen, by the reaction of a halogen substituted tetrahydrophthalic phthalic anhydride of the formula:

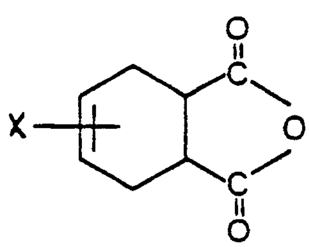

or a geminal dihalogen substituted hexahydrophthalic anhydride of the formula:

EP 0 334 049 A2

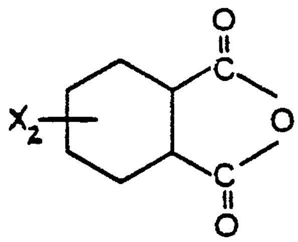

in a vapor or melt phase while mixed with a halogen gas and in the presence of an activated carbon catalyst at an elevated temperature.

# IMPROVED DEHYDROGENATION PROCEDURE

This invention is concerned with the process for the aromatization of a cyclic compound to form a fully aromatic ring structure. More particularly, it relates to a process for producing a halogen substituted phthalic anhydride from a halogen substituted tetrahydrophthalic anhydride or from a dihalogen substituted hexahydrophthalic anhydride.

Substituted phthalic anhydrides are valuable raw materials for the synthesis of useful products. These anhydrides are utilized as intermediates in the synthesis of organic polymers, dyes, plasticizers and in other uses.

Procedures are known for preparing substituted phthalic anhydrides. U.S. Patent 4,560,772 discloses the reaction of 4-methyl-tetrahydrophthalic anhydride with excess sulfur and a catalytic amount of zinc oxide and 2-mercaptobenzothiazole to produce 4-methyl phthalic anhydride and hydrogen sulfide.

U.S. Patent 4,560,773 discloses a similar reaction between the electron rich 4-methyl-tetrahydrophthalic anhydride and bromine in the presence of a catalytic amount of an acid acceptor such as dimethylformamide or pyridine in the liquid phase.

U.S. Patent No. 4,709,056 discloses the dehydrohalogenation of dihalohexahydrophthalic anhydrides through the use of a basic alumina catalyst in a liquid phase to produce 4-fluoro-1,2,3,6-tetrahydrophthalic anhydride.

Ohkatou et al, J. Japan Petrol. Inst., 22, 164-9 (1979) discloses the dehydrogenation of hydrocarbons using an activated carbon bed to produce the corresponding olefins. The mechanism of the reaction using cyclohexane and cyclohexene were studied using a pressure flow technique.

Bergmann, J. Amer. Chem. Soc., 64, 176 (1942) discloses the aromatization of tetrahydrophthalic anhydride products of Diels-Alder reactions. The author discloses that dehydrogenation occurs when the tetrahydrophthalic anhydride product is boiled in nitrobenzene. However, it is further disclosed that dehydrogenation does not occur when p-bromonitrobenzene, p-chloronitrobenzene, or m-dinitrobenzene in xylene is employed. Moreover, it has been found that when the dihalohexahydrophthalic anhydrides of this publication are dehydrogenated in nitrobenzene, a portion of the nitrobenzene is reduced to aniline. The aniline reacts with the anhydride group of either the starting material or product to form imides and thus lower the yield of desired product.

Skvarchenko et al, Obshchei Khimiil, Vol. 30, No. 11, pp 3535-3541, (1960) disclose the aromatization of chlorosubstituted tetrahydrophthalic anhydride by heating with phosphorus pentoxide. In the aromatization process described however, decarboxylation also occurs with the formation of the corresponding chlorosubstituted benzene compound. The preparation of tetrahydrophthalic acids and anhydrides and various methods for dehydrogenation and aromatization thereof are reviewed by Skvarchenko in Russian Chemical Reviews, Nov. 1963, pp 571-589.

The aromatization of organic compounds has been shown by various techniques and the use of special catalysts seem to control the satisfactory conversion at useful product yields and conversion times. This study was undertaken to determine if a vapor state aromatization of halogen substituted materials such as halogenated tetrahydrophthalic anhydrides or dihalogenated hexahydrophthalic anhydrides could convert the material to a mono halogenated phthalic anhydride at adequate yields and within a reasonable time.

It is the principal object of the present invention to provide novel intermediate compounds which are useful and provide a commercially attractive synthetic route for the preparation of a halogen substituted phthalic anhydride from a halogen substituted tetrahydrophthalic anhydride, or from a dihalogen substituted hexahydrophthalic anhydride.

The reaction can be conducted either in the vapor phase or in a liquid (melted) state by the passage of a halogen through the reaction zone at an elevated temperature in the presence of an activated carbon as a catalyst. For the vapor phase reaction, the temperature is in the range of about 200 to 500 °C, preferably about 200 to 325 °C. For the liquid as melt phase reaction, the temperature is in the ranges of about 200 to 300 °C, preferably about 200 to 250 °C.

A specific example of the reaction employs 4-chlorotetrahydrophthalic anhydride as a starting material and yields 4-chlorophthalic anhydride as the end product in high yields:

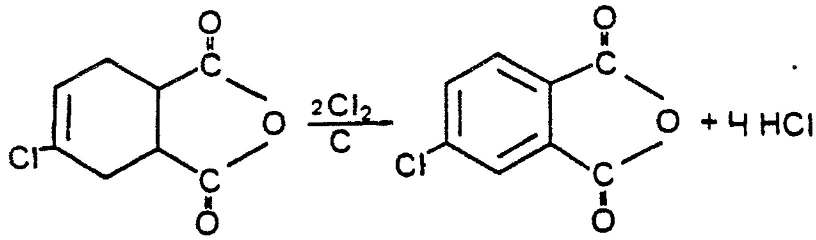

In the method of the invention the use of halogen gas decreases the reaction time and increases the yield of the product.

This invention is concerned with the aromatization of a cyclic compound to form a fully aromatized ring and specifically to convert a halogen substituted tetrahydrophthalic anhydride or a geminal dihalogenated hexahydrophthalic anhydride into a halogen substituted phthalic anhydride. The aromatization reaction occurs in either the vapor or liquid phase by the passage of halogen through the reaction zone in the presence of activated carbon as a catalyst.

The starting material employed in this invention can be prepared by various known preparation methods, and many such compounds as commercially available. Diels-Alder addition reaction of a maleic anhydride with a conjugated diene is a typical reaction which produces such an anhydride with a partially saturated six membered ring. Depending upon the desired product, the diene and/or the maleic anhydride can contain substituents to provide the starting material for this invention. The following halogen substituted partially saturated phthalic anhydrides can be employed but the invention is not limited to the following: 4-chloro-1,2,3,6-tetrahydrophthalic anhydride;
4-fluoro-1,2,3,6-tetrahydrophthalic anhydride;
4-bromo-1,2,3,6-tetrahydrophthalic anhydride;
4-chloro-1,2,5,6-tetrahydrophthalic anhydride;
4-fluoro-1,2,5,6-tetrahydrophthalic anhydride;
4-bromo-1,2,5,6-tetrahydrophthalic anhydride;
4,4-difluorohexahydrophthalic anhydride;
4,4-dichlorohexahydrophthalic anhydride;
4,4-dibromohexahydrophthalic anhydride;
3-chloro-1,2,5,6-tetrahydrophthalic anhydride;
3-fluoro-1,2,5,6-tetrahydrophthalic anhydride;
3-bromo-1,2,5,6-tetrahydrophthalic anhydride;
3,3-difluorohexahydrophthalic anhydride;
3,3-dichlorohexahydrophthalic anhydride;
3,3-dibromohexahydrophthalic anhydride; the corresponding iodide derivatives can also be included.

The catalyst effective in this process may be any commercially available activated charcoal. Conveniently used forms are granular activated charcoals such as those available from Calgon Corporation, Pittsburgh, Pa., designated Calgon F-300 (coal based); Calgon PCB (coconut-shell based); Calgon APC (coal based), Norit MRX (peat based) and Norit KB activated carbon (peat based) available from American Norit Company, Jacksonville, Fla.; Carborundum GAC 40; Aldrich activated carbon (Cat. No. 24,223-3) available from Aldrich Chemical Co., Milwaukee, Wisc., or similar granular forms suitable for passing organic vapors through them. Alternatively, powdered forms of activated charcoal may be used.

The aromatization reaction can be conducted in the vapor phase or under liquid conditions. The vapor phase reaction will be discussed first.

The vapor phase reaction is conducted using a metallic tube, preferably of a nickel base which is heated to a temperature to assist in the vaporization of the components. Optionally the reaction can be run under vacuum. A portion of the tube contains activated carbon. The procedure involves heating, for example, 4-chloro-1,2,3,6-tetrahydrophthalic anhydride until it melts and then with the aid of a pump, is pumped into the heated reaction tube where it vaporizes. Chlorine gas is also passed into the heated reaction tube. The two gases mix as they pass through the tube entering the region of the tube wherein the activated charcoal was placed. The reaction occurs in this region and the products are vented from the tube into a cooler container which allows the collection of the 4-chlorophthalic anhydride and the HCl produced is trapped in an aqueous media.

If the liquid or melt phase system is used, the procedure involves the melting, for example of 4-chlorotetrahydrophthalic anhydride, addition of activated carbon as catalyst and the passage of chlorine gas into a reactor containing a stirrer and outlet traps to contain the HCl being produced.

Two moles of chlorine are required for each mole of starting material. The major side product is anhydrous HCl. A slight excess of chlorine can be used to facilitate the reaction in either the vapor or liquid phase.

In the above description, the halogen reactant was identical to the halogen on the substituted tetrahydrophthalic anhydride starting material, but this need not be the case. Any halogen, excluding fluorine, will function as a hydrogen acceptor. If a different halogen is used, small amounts of mixed halogen side products can be produced. The major product will be the phthalic anhydride substituted with the same halogen as the starting material.

A solvent can be used in the reaction, but is generally not preferred. When a solvent is used, the starting material is dissolved in the solvent prior to introduction of the starting material to the reactor. In the vapor phase reaction, a lower boiling solvent is preferred, such as toluene or vinyl acetate. In the melt or liquid phase reaction, a higher boiling solvent such as 1,2,4-trichlorobenzene is preferred.

## Example I

A U-shaped nickel reactor tube, 1-inch ID, 30-inches long on each side, was wrapped with heating tapes. The input leg is used as the vaporizer; the outlet leg is packed with carbon and used as the reactor. The outlet end of the tube was packed with 146 grams of activated carbon (Calgon F-300 manufactured by Calgon Corporation, Pittsburgh, Pennsylvania). Placed within the carbon bed are thermocouples to measure the temperature. At the feed end of the U-shaped reaction tube are two openings, one for the chlorine gas feed and the other for the raw material feed. To the outlet of the reactor is placed a series of receivers and traps to collect the product and entrap the anhydrous HCl produced. A quantity of 4-chlorotetraphthalic anhydride was placed in a flask and heated. The melted material was transferred, in a steady flow by means of a pump, through heated lines to the inlet of the reactor tube. Prior to starting, the pressure was reduced to 50 mm of Hg to lower the temperature of vaporization. The raw material was vaporized at 250°C. The reaction temperature was 240 to 250°C. Chlorine flow was 230 ml/min, and the organic flow was 0.67 grams/minute. The ratio of the flow of chlorine and the flow of organic was set at 2 moles of chlorine per mole of organic. The reaction was conducted under these temperatures and conditions and a product of 4-chlorophthalic anhydride was obtained at an 80% yield and 90% purity.

## Example II

A mixture of 20 grams of 4-chlorotetrahydrophthalic anhydride and 5 grams of activated carbon, (NORIT KB) was placed in a reactor containing a gas inlet tube, a stirrer and condenser and an outlet connected to a trap. The reactor was heated to 250°C and a continuous flow of chlorine gas was passed through the liquid for 1.5 hours. Product began to collect in the trap, the temperature was reduced to 220°C, and stirring was continued for an additional hour. The yield of 4-chlorophthalic anhydride was 60% and analysis of the reaction mixture showed no starting material being present. The purity of the product was 60%.

## EXAMPLE III

The procedure of Example I was repeated except that the feed was 4,4-difluorohexahydrophthalic anhydride, the vapor temperature was 240°C, reaction temperature was 225°C, the organic rate was 0.5 grams/minute, and the chlorine rate was 183 ml/min. The product, 4-fluorophthalic anhydride, was produced in 80% yield and 90% purity.

## EXAMPLE IV

The procedure of Example II was repeated except that the halogen was liquid bromine (11.08 ml), 19.9 grams of the organic reactant, and 2.01 grams DARCO KB activated carbon was used. The reactor was heated to 200°C and the bromine dropped in over a period of one hour. The product yield was 13% 4-chlorophthalic anhydride and 2% 4-bromophthalic anhydride.

While the invention has been described in connection with specific embodiments thereof, it will be understood that it is capable of further modification, and this application is intended to cover any variation,

uses, or adaptations of the invention following, in general, the principles of the invention and including such departures from the present disclosure as come within known or customary practice in the art to which the invention pertains and as may be applied to the essential features hereinbefore set forth, as fall within the scope of the invention.

## Claims

1. A process for the preparation of halogen substituted phthalic anhydride of the formula:

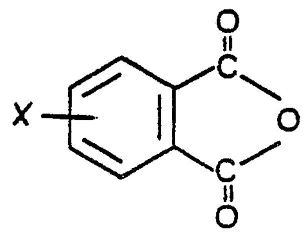

where X is a halogen, by reacting either a halogen substituted tetrahydrophthalic anhydride of the formula:

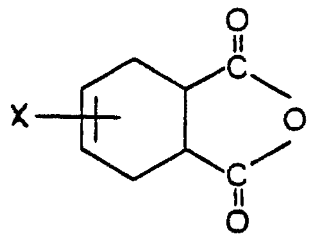

or a geminal dihalogen substituted hexahydrophthalic anhydride of the formula:

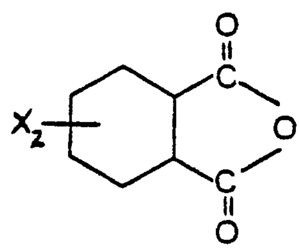

admixed with a halogen in the presence of an activated carbon catalyst
a) in the vapor phase at a temperature of 200-500° C; or
b) in a liquid phase at a temperature of 200-300° C.

2. The process of Claim 1, wherein the tetrahydrophthalic anhydride is 4-chlorotetrahydrophthalic anhydride.

3. The process of Claim 1, wherein the tetrahydrophthalic anhydride is 3-chlorotetrahydrophthalic anhydride.

4. A process of Claim 1, wherein the phthalic anhydride product is 4-chlorophthalic anhydride.

5. A process of Claim 1, wherein the phthalic anhydride product is 3-chlorophthalic anhydride.

6. A process of Claim 1, wherein the tetrahydrophthalic anhydride is 4-fluorotetrahydrophthalic anhydride.

7. A process of Claim 1, wherein the phthalic anhydride product is 4-fluorophthalic anhydride.

8. A process of Claim 1, wherein the tetrahydrophthalic anhydride is 4-bromotetrahydrophthalic anhydride.

9. A process of Claim 1, wherein the phthalic anhydride product is 4-bromophthalic anhydride.

10. A process of Claim 1, wherein the hexahydrophthalic anhydride is 4,4-difluorohexahydrophthalic anhydride.

11. A process of Claim 1, wherein the reaction is conducted in the vapor phase in the range of 200-325 °C.

12. A process of Claim 1, wherein the reaction is conducted in a liquid phase in the range of 200-250 °C.